# EUROPEAN PATENT APPLICATION

(11) **EP 4 817 667 A2**
(43) Date of publication of application: **07.10.2026**
(21) Application number: 26192356.9
(22) Date of filing: 22.11.2022
(51) Int. Cl.: A61M 25/00

(54) **SHEATH FOR IMPLANTING AN ELECTRODE IN THE SEPTUM OF A HUMAN HEART**

(30) Priority: 03.12.2021 EP 21212175
(62) Document number(s) of the earlier application(s) in accordance with Art. 76 EPC: 22821447.4 / 4 440 684
(71) Applicant: BIOTRONIK SE & Co. KG, 12359 Berlin (DE)
(72) Inventor: GRAUHAN, Ole, 14163 Berlin (DE); BOCK, Ralf, 12051 Berlin (DE); TROEGER, Uwe, 12351 Berlin (DE)
(74) Representative: Biotronik Corporate Services SE

(57) **Abstract**

A sheath (1) for implanting an electrode in the septum of a human heart, comprising an elongated sheath shaft (10) which delimits a lumen (L). The sheath shaft (10) comprises a preshaped distal end portion (101), wherein a curvature of the distal end portion (101) varies along the distal end portion (101).

## Description

The invention relates to a sheath for implanting an electrode in the septum of a human heart.

It is known to insert sheaths into the human body during the implantation of medical devices. These types of sheaths generally have an elongated sheath shaft (e.g., in the form of a silicone tube), which defines at least one lumen, wherein the implantable medical device may be pushed forward through the lumen to an implantation location in the body of the patient. In other words, these types of sheaths may function as delivery catheters for an implant.

For example, electrode leads of pacemaker systems are often guided to an implantation location in a cardiac chamber by means of this type of sheath. In particular, this may occur, e.g., in the context of an implantation of a pacemaker electrode for His bundle stimulation in the septum of the heart.

It is thereby generally desirable to configure the sheath so that it supports a positioning and anchoring of the electrode at the suitable implantation location as best as possible.

US 7,647,124 B2 and US 7,729,782 B2 each disclose embodiments of a delivery catheter, which is usable for inserting an electrode lead into the right atrium in close proximity to a His bundle. The delivery catheter comprises a proximal portion and a generally hook-shaped distal portion. The distal portion comprises two curved portions, substantially coplanar with the proximal portion, and with different curvatures. A linear connecting segment may thereby be provided between the two curved portions. In addition, the distal portion comprises a linearly distal end portion with a catheter tip.

These types of solutions, which rely on separate, differently shaped curved portions of a distal portion of the introducer sheath, are disadvantageous, in that they are comparatively sensitive to kinking due to the discontinuous course of the introducer sheath. This means that the introducer sheath buckles easily under load at the transition points between the the different curved portions, which may, e.g., limit the possibilities of a load transfer by means of the distal portion during the implantation.

The object of the present invention is to propose an improved sheath, in particular with respect to form stability, which supports a positioning and anchoring of an electrode in the septum of a human heart to the greatest extent. Furthermore, a method for implanting an electrode in the septum of a human heart by means of such a sheath is specified.

The subject matters of the independent claims are proposed starting from these points. Features of some embodiments are specified in the subclaims. The features of the subclaims may be combined with one another to form further embodiments, insofar as nothing against this is specifically specified.

According to a first aspect, the problem is solved by a sheath for implanting an electrode in the septum of a human heart. The sheath comprises an elongated sheath shaft which delimits a lumen. The sheath shaft has a flexible distal end portion, wherein the distal end portion is preshaped in such a way that it describes a helical curve. This means that the sheath shaft, including the lumen located therein, describes a helical curve.

A helical curve is to be understood as at least one portion of a three-dimensional curve which winds continuously around about a helix axis and thereby also has a component along the helix axis. It is thus, e.g., a three-dimensional spiral, which winds out of the plane like a type of snail shell. The helical curve may be, e.g., a portion of a three-dimensional spiral whose radius varies (and e.g. continuously increases along the helix axis).

Due to the helically curved configuration of the distal end portion of the sheath shaft, discontinuities due to kink susceptibilities are prevented. In addition, it is facilitated that the sheath shaft (in portions) lies stably in a cardiac vein and simultaneously shapes itself in the cardiac chamber (in another, more distal portion), such that it may support an implantation of an electrode in the septum.

In one preferred embodiment, the distal end portion of the sheath shaft is preshaped in a way that a local radius of the helical curve continuously increases along the sheath shaft from the distal to the proximal.

In particular, the local radius may exponentially increase from the distal to the proximal. For example, the helical curve may be described in polar coordinates in a top view along the helix axis, about which the helical curve winds, wherein a distal end of the sheath shaft is located at the origin of the coordinate system. In this case, the helical curve may be parameterized by a polar angle passed through, starting from the origin, wherein the local radius of the helical curve increases exponentially to the polar angle passed through.

It should be remembered that a local gradient along the helical curve may be variable (relative to the helix axis). The local gradient of the helical curve preferably increases from the proximal to the distal. Thus, the distal end may align, e.g., largely in the direction of the helix axis of the helical curve. For example, the distal end may face perpendicularly to the septum of the heart during the implantation of the electrode within the cardiac chamber.

According to one embodiment, the helical curve winds about a helix axis and thereby passes through an angle of a maximum of 340°. For example, in a top view along the helix axis, about which the helical curve winds, the helical curve may be described in polar coordinates, wherein a distal end of the sheath shaft is located at the origin of the coordinate system. In this case, a polar angle interval of e.g., 0 to a maximum of 340° may suffice to trace the entire helical curve of the distal end portion. Visually, this means that the helical curve according to this embodiment describes almost a maximum of a complete circle (namely at least 20° less than a complete circle) in the top view.

According to another embodiment, the angle passed through lies, in contrast, in the range from 4 rad to 20 rad. This embodiment thus visually allows that the distal end portion of the sheath shaft winds multiple times (up to 3 times) completely around the helix axis.

By indicating that the distal end portion is flexible, it should be expressed that the distal end portion is elastically malleable, wherein the distal end portion is preshaped in such a way that is adopts the helically curved shape at least in an unloaded (non-deformed) state).

The sheath shaft may consist, e.g., at least partially of a relatively flexible plastic material, like silicone. For example, such a sheath shaft may be produced in a so-called reflow process, in which one or more tube segments made from plastic are initially applied onto at least one wire. The at least one wire is subsequently removed so that at least one lumen remains.

It is also within the context of the invention that the sheath shaft has a (not necessarily monotonously) decreasing stiffness from the proximal to the distal. In other words, the sheath shaft may become increasingly softer from the proximal to the distal. In particular, the stiffness may monotonously decrease from the proximal to the distal. Due to the decrease in the stiffness of the sheath shaft from proximal to distal, the sheath shaft in the proximal portion may guarantee a good maneuverability and simultaneously be sufficiently flexible in the distal portion in order to be able to adapt well to the vascular tree.

The sheath shaft may comprise, e.g., multiple segments of different stiffness. The different stiffnesses of the individual segments may thereby be achieved, e.g., through tube segments made of materials of different hardnesses, by which means the stiffness may be varied.

According to one advantageous embodiment, the sheath shaft may have a soft tip at a distal end. This means that a tip at the distal end is particularly soft (i.e., is less stiff) in comparison to other portions of the sheath shaft and in particular in comparison to other portions of the distal end portion. This may be an atraumatic tip, which prevents injuries to the vessels or other tissues due to its soft configuration.

A second aspect relates to a method for implanting an electrode in the septum of a human heart. The method comprises the following steps:
- Providing a sheath according to the first aspect;
- Inserting the sheath into the body of the patient and pushing the distal end portion of the sheath shaft forward through a cardiac vein so that a curved portion of the distal end portion lies on a cardiac chamber wall (e.g., in the atrium) and the distal end faces in the direction of the septum within a cardiac chamber;
- Pushing an electrode forward through the lumen; and
- Fixing the electrode in the septum.

The electrode may be, in particular, a pacemaker electrode, which is implanted by means of the method at a location suitable for His bundle stimulation. During the implantation (e.g., in the atrium), a curved portion lying on the cardiac chamber wall may thereby produce particularly stable working conditions, in that it effectively supports the distal end even when the heart is moving. Thus, the forces required during the implantation of the electrode may be reliably applied perpendicularly to the septum.

To introduce the sheath into the vascular tree, a dilator is initially inserted into the lumen, by which means a curvature of the distal end portion is reduced. After removing the dilator, the distal end portion then adopts the preshaped helically curved shape, insofar as the surrounding tissues, in particular a surrounding cardiac vein like the superior vena cava (SVC) allow this.

For example, a preshaped proximal subsection of the distal end portion, which is itself curved, may be pulled comparatively straight through the course of the cardiac vein and may thus be held under a certain tension. Each subsection of the sheath body may lie particularly stably in the SCV during the implantation due to this, and support the more distally lying subsections of the distal end portion.

The sheath is preferably a slittable sheath. After the implantation, the sheath shaft may be slit open using a suitable slitter tool, withdrawn, and removed from the body of the patient.

Embodiments of the first aspect of the invention may be analogously used for the second aspect of the invention and vice versa.

Additional advantages and embodiments of the present invention are to be subsequently described with reference to the figures. As shown in:
- Fig. 1: schematically and by way of example, a preshaped distal end portion of a sheath shaft according to one or more embodiments in a side view;
- Fig. 2: the distal end portion of the sheath shaft from figure 1 in a top view;
- Fig. 3: schematically and by way of example, a dilator for use when introducing a sheath according to one or more embodiments; and
- Fig. 4: schematically and by way of example, a sheath according to one or more embodiments, with and without the dilator inserted into the lumen.

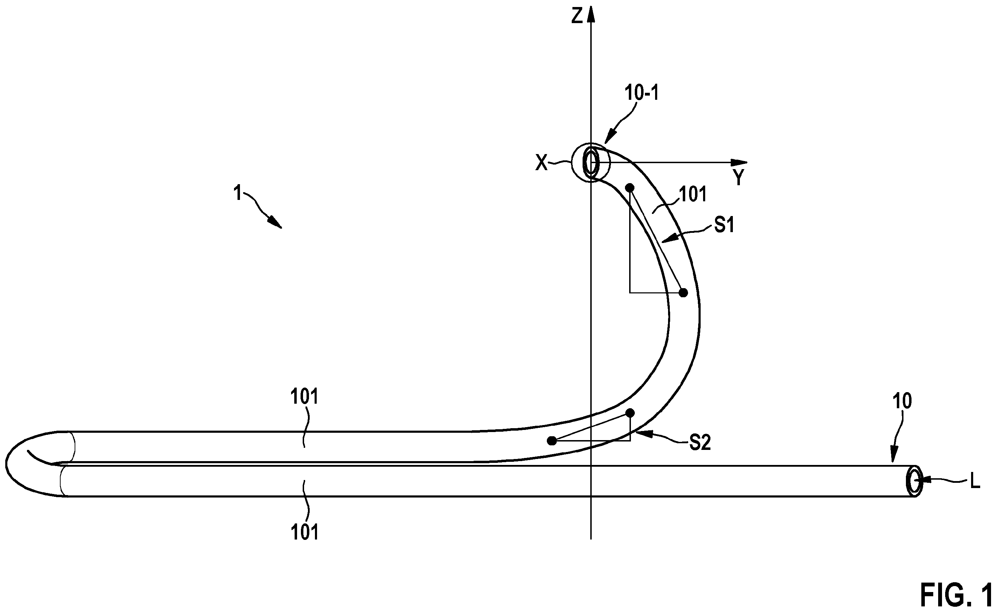

Figure 1 schematically and by way of example shows a distal end portion 101 of an elongated sheath shaft 10 according to one or more embodiments of a sheath 1 according to the invention. Sheath shaft 10 is depicted as truncated on the right (towards the proximal) so that it is visible that sheath shaft 10 defines a lumen L.

Through lumen L, a pacemaker electrode, e.g., may be pushed forward to an implantation site in the heart of a patient, after which a distal end 10-1 of sheath shaft 10 is placed in close proximity to the implantation site (e.g. in close proximity to the septum in the area of a His bundle).

Distal end portion 101 of sheath shaft 10 is preshaped in a way that it, together with lumen L configured therein, describes a three-dimensional helical curve.

In Figure 1, distal end portion 101 is shown in a side view. The helical curve, which distal end portion 101 follows, thereby winds perpendicularly about vertically extending helix axis Z.

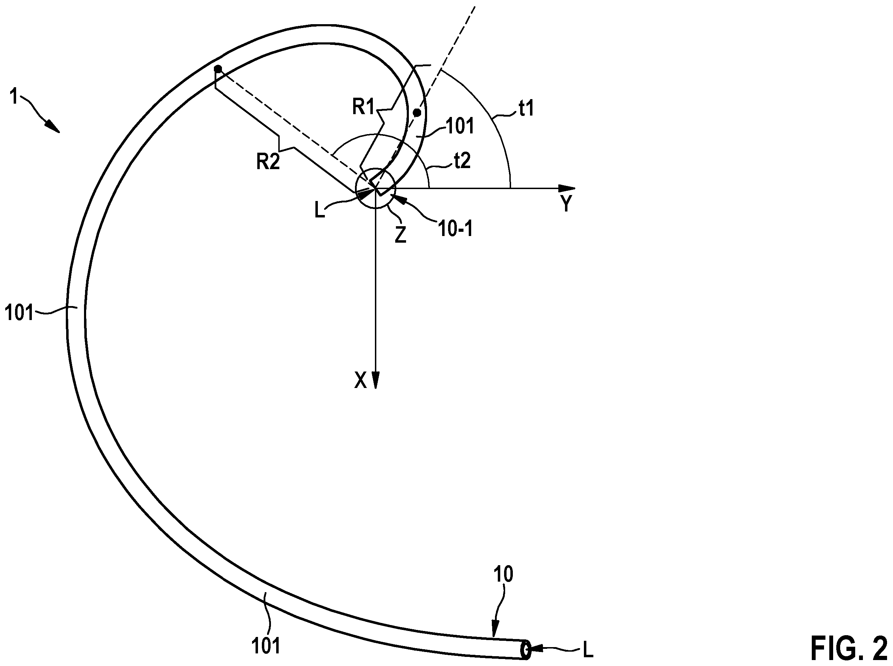

Figure 2 shows the helically curved shape of distal end portion 101 in a top view along helix axis Z, i.e., in a projection on a plane XY extending perpendicular to helix axis Z. It is clear in the top view that a local radius R1, R2 of the helical curve continuously increases along sheath shaft 10 in the direction from distal to proximal in the present embodiment. For illustration, two local radii R1, R2 are marked in Figure 2 by way of example, wherein second local radius R2, which lies more proximal than first local radius R1, is visibly greater than first radius R1.

The different local radii R1 and R2 further show that the curvature of distal end portion 101 varies along distal end portion 101. Accordingly, different portions of distal end portion 101 may exhibit different local curvatures.

It may be provided, e.g., that the local radius increases exponentially from distal to proximal. For example, the helical curve may be described in the XY plane in polar coordinates, wherein a distal end of the sheath shaft is located in the origin of the coordinate system, as Figure 2 illustrates. In this case, the helical curve may be parameterized, starting from the origin, by a polar angle t passed through. Corresponding angles t1 or t2 are assigned to first local radius R1 and to second local radius R2 in this parameterization, wherein second angle t2 is greater than first angle t1, as the parameterization is selected such that the helical curve is traced at an increasing angle t in the direction from distal to proximal.

Local radius R1, R2 of the helical curve may increase, e.g., exponentially with polar angle t passed through. This may be expressed, for example, in a parameterization of the X and Y components corresponding to two parameter equations for X(t) and Y(t) of the following type: $X \left(t\right) = - {k}_{1} exp \left({k}_{2}t\right) sin \left(t\right)$ $Y \left(t\right) = {k}_{1} exp \left({k}_{2}t\right) cos \left(t\right)$ where *k*₁ and *k*₂ are constants.

The side view according to Figure 1 shows that a local gradient S1, S2 (relative to helix axis Z) may also be variable along the helical curve. The local gradient of the helical curve preferably increases in the direction from proximal to distal. To illustrate, two local gradients (or corresponding gradient triangles) S1, S2 are marked by way of example. First local gradient S1, which lies more distal than second local gradient S2, is significantly greater than second local gradient S2. Sheath shaft 10 thus turns increasing out of the XY plane toward the distal end and faces increasingly in the direction of helix axis Z.

The different local gradients S1 and S2 further show that different portions of distal end portion 101 may exhibit different spatial orientations along distal end portion 101.

Accordingly, distal end 10-1 may exhibit an orientation different from the orientation of more proximal portions of distal end portion 101.

Due to a coordinated variation of the local gradient and the local radius, distal end 10-1 of sheath shaft 10 may, for example, face substantially perpendicular to the septum during the implantation of the electrode within the cardiac chamber, while curved preshaped subsections of distal end portion 101, lying more proximal, are supported on the wall of the atrium and/or in an end portion of a cardiac vein (in particular the SVC). By this means, a particularly stable working position may be established for the implantation of the electrode in the septum.

This may be expressed, for example, in a parameterization of the Z component corresponding to the parameter equation for Z(t) of the following type: $Z \left(t\right) = \frac{- {k}_{3}}{- {k}_{4} + {k}_{5} t} sin \left({k}_{6}+{k}_{7}t\right)$ where *k*₃, *k*₄, *k*₅, *k*₆, and *k*₇ are constants. By way of example, values for constants *kᵢ* are indicated: *k*₁ = 5, *k*₂ = 0,8, *k*₃ = 25, *k*₄ = 2, *k*₅ = 0,1, *k*₆ = 2,5, *k*₇ = 0,5. The configuration of sheath shaft 10 in distal end portion 101 may correspondingly be adapted to individual requirements by varying of constants *kᵢ* of the parameter equations. For the indicated constants *kᵢ*, polar angle t passes through, for example, values in the range from -1.658 to 4.323. These values for t correspond to an angle range of -95° to +250°. The end of the parameter equations for values of t in the range of -1.658 thereby describes an area of sheath shaft 10 which faces in the direction of distal end 10-1 of sheath shaft 10, and the end of the parameter equations for values of t in the range of 4.363 describe an area of sheath shaft 10 which faces in the direction of proximal end 10-2 of sheath shaft 10.

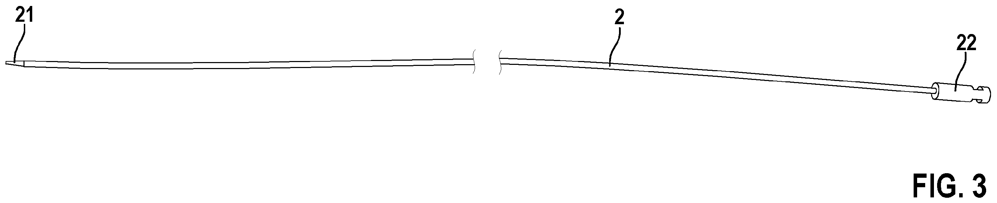

To insert sheath 1 into the blood vessel system, a dilator 2 may be initially inserted into lumen L. Such a dilator 2 is depicted by way of example in Figure 3. Dilator 2 has an elongated, comparatively stiff body with a slightly conically extending distal dilator tip 21. The dilator 2 has a handle 22 at a proximal end.

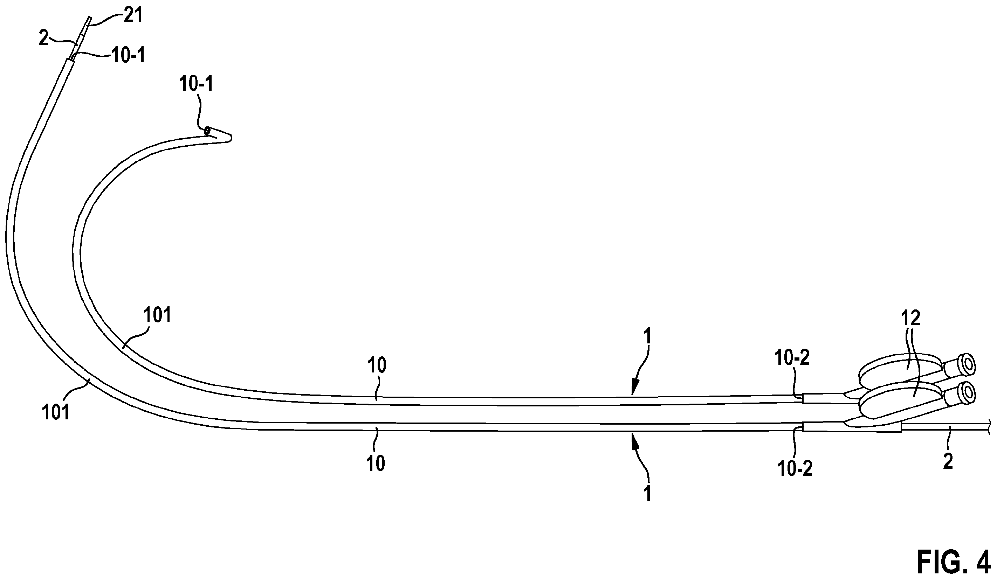

Figure 4 shows two sheaths 1 of the same type, wherein a dilator 2 of the type depicted in Figure 3 is inserted into lumen L of (only) lower sheath 1. In a comparison of the two sheaths 1, it is distinctly clear that comparatively stiff dilator 2 strongly reduces the curvature of preshaped distal end portion 101, in particular in the area of distal end 10-1.

As can further be seen in the projection of Fig. 4, different portions of distal end portion 101 may exhibit different apparent curvatures. In particular, a portion having a comparatively smaller apparent curvature may be located between portions having greater apparent curvatures

For example, sheath 1, in the state depicted below in Figure 4, i.e., ?? including inserted dilator 2, may be inserted into the body of the patient and distal end portion 101 may thereby be pushed forward through a cardiac vein (e.g. the SVC). Dilator 2 may then be withdrawn. After the removal of dilator 2 from lumen L, distal end portion 101 adopts its preshaped, helically curved configuration, insofar as the surrounding tissue, in particular a surrounding cardiac vein, e.g., the SVC, allows this.

For example, a preshaped proximal subsection of distal end portion 101, which is itself curved, may be pulled comparatively straight by the course of the cardiac vein and thus held under a certain tension. Due to this, that subsection of sheath shaft 10 may lie particularly stably in the cardiac vein during the implantation, i.e., when an electrode is pushed forward through lumen L, and support more distal lying subsections of distal end portion 101.

As a whole, the helically curved shape of distal end portion 101 allows a curved portion of distal end portion 101 to lie on a (e.g., atrial) cardiac chamber wall while distal end 10-1 faces in the direction of the septum within the cardiac chamber. For example, a force directed substantially perpendicular to the septum may thus be effectively exerted by means of the electrode.

In the embodiment according to Figure 4, a connector 12 is applied on a proximal end 10-2 of sheath shaft 10. Connector 12 may have, in addition to an opening to insert the electrode, e.g., a flushing connection and/or a hemostatic valve.

Sheath shaft 10 may have a decreasing stiffness (not necessarily monotonous) in the direction from proximal to distal, i.e., becoming increasingly softer here from proximal to distal. By this means, sheath shaft 10 may guarantee good maneuverability in the proximal portion and may simultaneously be sufficiently flexible in distal portion 101 in order to be able to adapt well to the vascular tree. For example, the stiffness may monotonously decrease from proximal to distal.

Sheath shaft 10 may have a soft tip at distal end 10-1. This means that a tip at distal end 10-1 is particularly soft (i.e., less stiff) in comparison to other portions of sheath shaft 10 - and in particular also in comparison to other portions of distal end portion 101. This may thus be an atraumatic tip, which prevents injury to the veins or other tissues during the pushing forward of the sheath due to its soft configuration.

In general, distal end portion 101 may be characterized by a variation of local radius and/or local gradient along its length, resulting in different local curvatures and/or different spatial orientations along distal end portion 101.

### List of reference numerals

- 1: Sheath
- 10: Sheath shaft
- 10-1: Distal end
- 10-2: Proximal end
- 101: Distal end portion
- 12: Connector

- 2: Dilator
- 21: Dilator tip
- 22: Handle

- L: Lumen
- R1, R2: Local radii
- S1, S2: Local gradients
- t, t1, t2: Angle
- X, Y, Z: Spatial directions

## Claims

1. A sheath (1) for implanting an electrode in the septum of a human heart, comprising an elongated sheath shaft (10) which delimits a lumen (L),
**characterized in that**
the sheath shaft (10) comprises a preshaped distal end portion (101), wherein a curvature of the distal end portion (101) varies along the distal end portion (101).

2. The sheath (1) according to claim 1, **characterized in that** the distal end portion (101) comprises portions having different local radii (R1, R2).

3. The sheath (1) according to claim 2, **characterized in that** the distal end portion (101) follows a three-dimensional curve.

4. The sheath (1) according to one of the preceding claims, **characterized in that** the distal end portion (101) comprises portions having different local gradients (S1, S2).

5. The sheath (1) according to claim 4, **characterized in that** a local gradient (S1, S2) increases in a direction from proximal to distal.

6. The sheath (1) according to any one of the preceding claims, **characterized in that** the distal end portion (101) comprises a portion having a smaller local curvature than another portion of the distal end portion (101).

7. The sheath (1) according to any one of the preceding claims, **characterized in that** the distal end (10-1) is oriented differently from an adjacent portion of the distal end portion (101).

8. The sheath (1) according to claim 7, **characterized in that** the distal end (10-1) is inclined relative to said adjacent portion.

9. The sheath (1) according to any one of the preceding claims, **characterized in that** the distal end portion (101) exhibits a plurality of changes in curvature.

10. The sheath (1) according to any one of the preceding claims, **characterized in that** at least a part of the distal end portion (101) follows a helical curve.

11. The sheath (1) according to claim 10, **characterized in that** a local radius (R1, R2) of the helical curve increases from distal to proximal.

12. The sheath (1) according to claim 11, **characterized in that** the local radius (R1, R2) increases exponentially from distal to proximal.

13. The sheath (1) according to claim 6, **characterized in that** the portion having the smaller local curvature is arranged between portions having greater local curvature when viewed in projection.

14. The sheath (1) according to claim 13, **characterized in that** the portion having the smaller local curvature appears substantially straight when viewed in projection.

15. The sheath (1) according to claim 7 or 8, **characterized in that** the distal end (10-1) forms a bend relative to said adjacent portion when viewed in projection.
